# EUROPÄISCHE PATENTSCHRIFT

(11) **EP 1 614 758 B1**
(45) Veröffentlichungstag und Bekanntmachung des Hinweises auf die Patenterteilung: **21.11.2007**
(21) Anmeldenummer: 05012272.0
(22) Anmeldetag: 08.06.2005
(51) Int. Cl.: C21C 5/46, F27B 3/28, F27D 19/00, F27D 21/00

(54) **Vorrichtung zur Durchführung von Messungen und/oder Probenahmen in Metallschmelzen**
Measuring lance for measuring operations and/or sampling in molten metals
Lance de mesure pour faire des mesures et/ou prélèvement d'échantillons dans les métaux en fusion

(30) Priorität: 16.06.2004 DE 102004028789
(43) Veröffentlichungstag der Anmeldung: 11.01.2006
(73) Patentinhaber: Heraeus Electro-Nite International N.V., 3530 Houthalen (BE)
(72) Erfinder: Neyens, Guido Jacobus, 3680 Opoeteren (BE); Bell, Shaun Andrew, Zelionople Pennsylvania 16063 (US)
(74) Vertreter: Kühn, Hans-Christian

(56) Entgegenhaltungen:
- EP-A- 0 069 433
- JP-A- 53 079 703

## Beschreibung

Die Erfindung betrifft eine Vorrichtung zur Durchführung von Messungen und/oder Probennahmen in Metallschmelzen mit einer Sublanze, die einen Sublanzenkörper aufweist, an dessen einem Ende ein Lanzenhalter zur Aufnahme einer Eintauchsonde angeordnet ist.

Derartige Vorrichtungen sind den Fachleuten hinreichend bekannt. Sie dienen der Messung bzw. Probennahme in Metallschmelzen. Derartige Probennahmen werden zum Teil automatisiert, wobei eine Sublanze in einen Schmelzbehälter eingetaucht wird zur Messung, danach wird die an der Sublanze angeordnete Eintauchsonde, da sie verbraucht ist, verworfen und eine neue Eintauchsonde auf die Sublanze aufgesteckt. Um diesen Vorgang zu automatisieren, muss die Sublanze exakt über einen Sondendepot positionierbar sein. Inder Praxis zeigt es sich jedoch, dass wegen der Belastungen, denen eine Sublanze während des Gebrauchs ausgesetzt ist, diese Sublanzen geringfügig verbogen werden, so dass der Lanzenhalter nicht mehr exakt über einer Eintauchsonde platzierbar ist und diese nicht einwandfrei aufnehmen kann. Die auf die Sublanzen aufgesteckten Eintauchsonden weisen nicht exakt die gleiche Länge auf. Insbesondere der im Trägerrohr untergebrachte Kontaktteil ist für den Gegenkontakt in der Sublanze nicht stets in der gleichen Tiefe erreichbar. Dazu werden sich häufig Spritzer der Metallschmelze an Teilen der Sublanze anlagern, die für die Kontaktierung frei bleiben müssen, so dass ein einwandfreies Aufstecken der Eintauchsonden nicht möglich ist. Dadurch kann der gesamte Stahlherstellungsprozess gestört werden.

Sublanzen sind beispielsweise aus EP 69 433 bekannt. Hier wird versucht, der Verformung der Sublanze während des Betriebes durch Drehen der Sublanze entgegenzuwirken. Die Anordnung und Funktion von Sublanzen ist des weiteren in DE 43 06 332 A1 beschrieben. Hier ist auch der Wechselprozess der Probensonden offenbart. Eine weitere Sublanze ist aus EP 143 498 A2 bekannt. Die hier dargestellte Sublanze weist an Verbindungsstellen eine Abdichtung, beispielsweise einen Gummiring auf, der verhindert, dass in die Mechanik flüssiges Metall eindringen kann.

Der Erfindung liegt die Aufgabe zugrunde, die bekannten Sublanzen zu verbessern und insbesondere die Ausfallsicherheit im automatischen Betrieb zu erhöhen.
Die Aufgabe wird für die eingangs charakterisierte Erfindung dadurch gelöst, dass an dem Lanzenhalter ein Kontaktstück angeordnet ist zur Kontaktierung von Signalleitungen der Eintauchsonde, weiterhin dadurch, dass der Sublanzenkörper mit dem Lanzenhalter oder einem Teil davon beweglich verbunden ist und dass der Lanzenhalter mehrere gegeneinander bewegliche Teile aufweist, wobei das Kontaktstück beweglich zur Sublanze, insbesondere zum Sublanzenkörper, angeordnet ist. Die Sublanze wird auf das Oberteil der Lanzenhalterung fest aufgeschoben und vorzugsweise unbeweglich gehalten. Das untere Teil der Lanzenhalterung mit dem Kontaktstück ist beweglich, damit die Sonde in Kontakt gebracht werden kann mit dem Kontaktstück. Dadurch kann sich die Lanzenhalterung der Lage des bereit gestellten Sensors anpassen, so dass ein ausreichender Kontakt auch bei leicht verbogenen Bauteilen oder dann möglich ist, wenn Metallschmelze an der Sublanze anhaftet. Unterschiedliche Einstecktiefen der Sublanze in das Trägerrohr der Eintauchsonde werden auf diese Weise ebenfalls ausgeglichen. Vorzugsweise sind der Sublanzenkörper mit dem Lanzenhalter und/oder die Teile des Lanzenhalters in Achsrichtung und/oder radial beweglich angeordnet. Des weiteren ist es vorteilhaft, dass die axiale Beweglichkeit und die radiale Beweglichkeit durch voneinander verschiedene Paare von Verbindungsteilen realisiert sind, um eine höchstmögliche Flexibilität und Anpassungsfähigkeit zu erhalten. Gerade bei Anhaften von Metallschmelze an der Sublanze oder bei unterschiedlich angeordneten Kontaktteilen der Eintauchsonde ist eine axiale Beweglichkeit von Bedeutung, um die korrekte Kontaktierung der Eintauchsonde zu gewährleisten. Insbesondere ist es vorteilhaft, dass die Beweglichkeit durch zwischen starren Teilen angeordnete elastische Teile realisiert ist, wobei die elastischen Teile als Feder, beispielsweise als Schraubenfeder oder als elastischer Ring ausgebildet sein können. Des weiteren ist es zweckmäßig, dass ein Teil des Lanzenhalters eine Aufnahmebohrung aufweist, in der ein Zapfen eines zweiten Teils des Lanzenhalters axial beweglich angeordnet ist, wobei zwischen dem ersten und dem zweiten Teil des Lanzenhalters eine Schraubenfeder angeordnet ist. Dazu ist es von Vorteil, dass die Schraubenfeder zwischen einer an der Stirnseite oder an der Umfangsfläche des Zapfens angeordneten Anschlagsfläche und einer an oder in der Bohrung angeordneten zweiten Anschlagsfläche angeordnet ist. Die die Beweglichkeit gewährleistenden Bauteile sind dadurch selbst geschützt.

Zweckmäßigerweise weist ein Teil des Lanzenhalters eine Aufnahmebohrung auf, in der ein Zapfen eines zweiten Teils des Lanzenhalters radial beweglich angeordnet ist, wobei zwischen dem ersten und dem zweiten Teil des Lanzenhalters mindestens ein elastischer Ring angeordnet ist. Der elastische Ring kann vorteilhafterweise in radialer Richtung zwischen den beiden Teilen des Lanzenhalters angeordnet sein.

Nachfolgend wird ein Ausführungsbeispiel der Erfindung anhand einer Zeichnung beschrieben. In der Zeichnung zeigt:
- Fig. 1: einen Konverterofen mit einer Sublanze,
- Fig. 2: eine Sublanze mit Lanzenhalter und Eintauchsonde und
- Fig. 3: eine Lanzenhalterung im Detail.

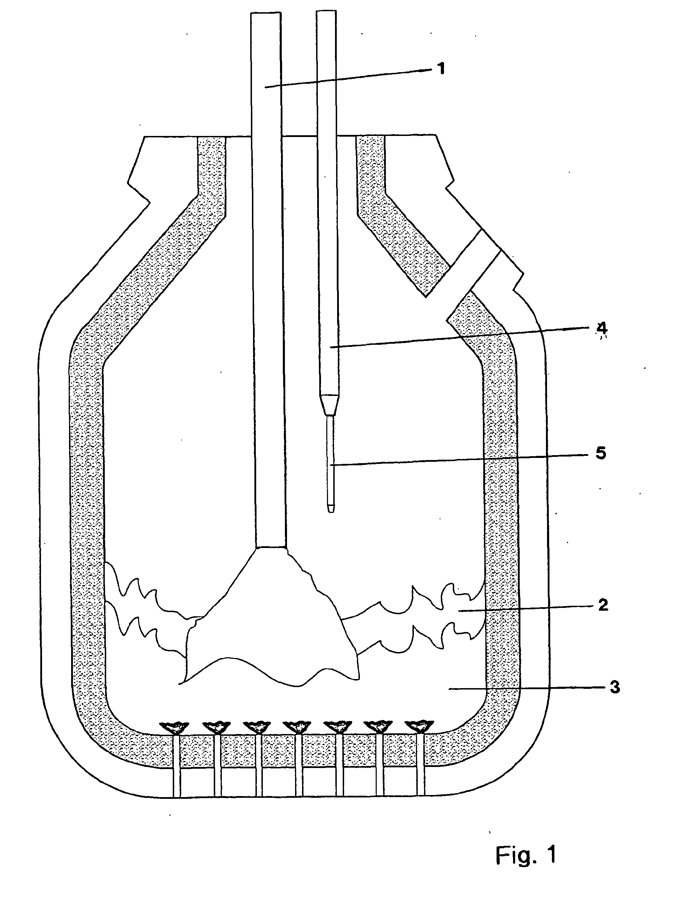

In dem in Figur 1 dargestellten Konverterofen ist eine Blaslanze 1 angeordnet, die Sauerstoff in die Schlacke- 2 bzw. Stahlschmelze 3 bläst. Daneben ist eine Sublanze 4 mit einer Eintauchsonde 5 angeordnet. Die Sublanze 4 fährt von oben in den Konverterofen hinein bis die Eintauchsonde 5 in die Stahlschmelze 3 eintaucht. Nach der Messung wird sie emporgezogen, die Eintauchsonde 5 ist zerstört. Wenn sie als Messsonde ausgelegt ist, ist die Messung während des Eintauchens in der Stahlschmelze 3 erfolgt. Eine in der Eintauchsonde 5 angeordnete Probenkammer wurde in der Stahlschmelze 3 gefüllt. Die Probenkammer wird der abgeworfenen Eintauchsonde 5 entnommen und die Probe kann ausgewertet werden. Für die nächste Messung wird eine weitere Eintauchsonde 5 aus einem Depot entnommen, in der Regel maschinell auf die Sublanze 4 aufgesteckt und zur Messung in den Konverterofen eingeführt.

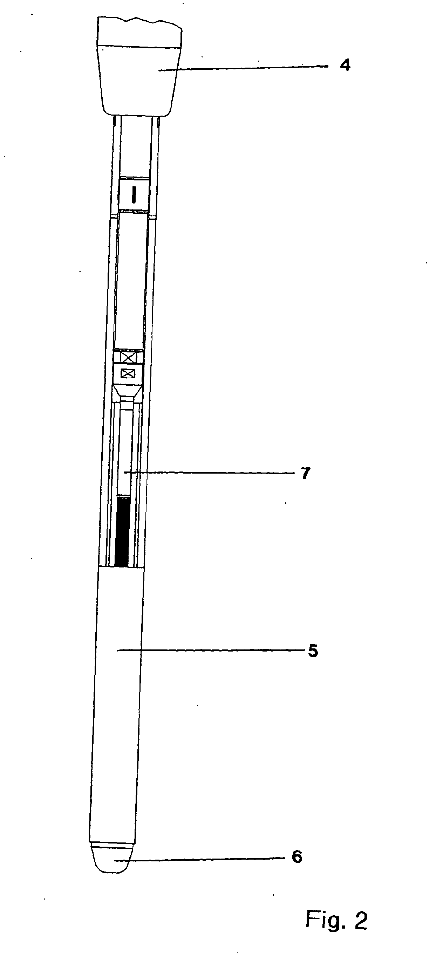

Figur 2 zeigt die am unteren Ende der Sublanze 4 angeordnete Eintauchsonde 5. Die Eintauchsonde 5 weist ein Eintauchende auf, welches gegen die auf der Stahlschmelze 3 aufliegende Schlackeschicht 2 durch eine Kappe 6 geschützt ist, die dem Sensor bzw. die Probenkammer erst nach Eintauchen in die Stahlschmelze 3 freigibt. Die Eintauchsonde 5 ist mittels des Lanzenhaltes an der Sublanze 4 befestigt. Mittels eines an dem Lanzenhalter angeordneten Kontaktstück 7 werden die Signalleitungen der Eintauchsonde 5 kontaktiert, so dass die Messsignale durch die Sublanze 4 hindurch zu einer Auswerteeinheit geführt werden können.

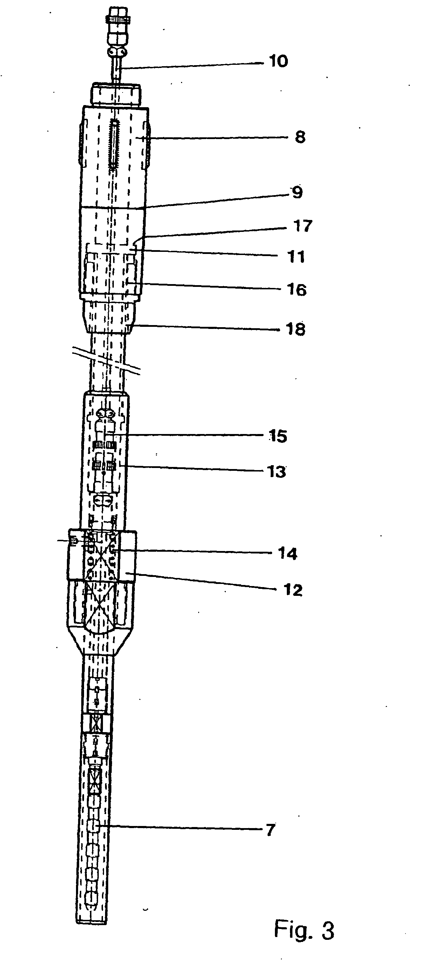

In Figur 3 ist der Lanzenhalter dargestellt. Der Lanzenhalter ist ein wiederbenutzbares Teil der Sublanze 4. Er dient zur Halterung der Eintauchsonde 5 und als Kontaktverbindung mit der Eintauchsonde 5. Der Lanzenhalter ist mit dem wassergekühlten Teil der Sublanze 4 verbunden. Die Wasserkühlung ist in den Figuren nicht näher erläutert. Sie ist aus dem Stand der Technik (beispielsweise EP 69 433) hinreichend bekannt. Der Lanzenhalter ist mit seinem oberen Teil 8 starr in der Sublanze 4 angeordnet und sein unterer Teil, beginnend etwa ab der Trennlinie 9 in der Eintauchsonde 5. Dabei stellt das Kontaktstück 7 die elektrische Kontaktierung mit den Signalleitungen der Eintauchsonde 5 sicher. Die Ableitung der elektrischen Signale und Weiterleitung an eine Mess- oder Auswertestation erfolgt durch das Lanzenkabel 10, welches am oberen Ende des Lanzenhalters angeordnet ist und durch die Sublanze 4 hindurchgeführt wird. Im oberen Bereich des Lanzenhalters ist ein Gummiring 11 angeordnet. Der Gummiring 11 ermöglicht sowohl eine radiale als auch eine axiale Bewegung des unteren Teils des Lanzenhalters gegenüber dem Oberteil 8. Der Gummiring 11 ist mittels einer Schraube 16 gegen einen Anschlag 17 gehalten. Die Schraube 16 weist in axialer Richtung ein durchgehendes Loch 18 auf, das in Richtung Kontaktstück 7 konisch erweitert ist. Dadurch ist das Oberteil 8 des Lanzenhalters gegenüber dem unteren Teil radial und geringfügig auch axial beweglich. Statt eines Gummirings 11 kann auch eine Metallfeder, beispielsweise eine Schraubenfeder verwendet werden. Der untere, das Kontaktstück 7 aufweisende Teil des Lanzenhalters weist eine Dichtungshülse 12 auf, in die ein Führungsrohr 13 hineinragt. In Längsrichtung zwischen dem Führungsrohr 13 und einer inneren Anschlagsfläche der Dichtungshülse 12 ist eine Schraubenfeder 14 angeordnet. Dadurch ist eine Beweglichkeit des Kontaktstücks 7 mit der Dichtungshülse 12 längs des Führungsrohrs 13 gewährleistet. Diese Beweglichkeit sichert auch bei unterschiedlicher Länge verschiedener Eintauchsensoren 5, die auf den Lanzenhalter aufgesteckt werden, stets eine sicheren Kontakt zwischen den Signalleitungen des Eintauchsensors 5 und dem Kontaktstück 7. Ein sicherer Kontakt ist auch dann gewährleistet, wenn sich auf den Lanzenhalter Verunreinigungen, wie Metallschmelze oder Schlacke festgesetzt haben. Dies kann im oberen Teil des Lanzenhalters dort passieren, wo die Sublanze 4 und die Eintauchsonde 5 aneinander stoßen. Auch in einem solchen Fall der Verunreinigung ist durch die Feder 14 ein zuverlässiger Kontakt zwischen dem Kontaktstück 7 und den Signalleitungen der Eintauchsonde 5 gewährleistet. Die Feder 14 weist vorzugsweise eine Federspannung auf, die größer ist als die Aufsteckkraft des Kontaktstückes 7 auf den sogenannten Konnektor innerhalb der Eintauchsonde 5, mit dessen Hilfe die Signalleitungen mit dem Kontaktstück kontaktiert werden. Innerhalb des Führungsrohrs 13 können Kupplungselemente 15 vorgesehen sein, über die das Lanzenkabel 10 mit dem Kontaktstück 7 verbunden wird. Auf die gezeigte Weise ist das Kontaktstück 7 sowohl längs als auch radial beweglich zur Sublanze 4 angeordnet und kann daher mit den Signalleitungen der Eintauchsonde auch dann verbunden werden, wenn das untere, den Lanzenhalter tragende Ende der Sublanze 4 leicht gebogen ist. Ebenso ist eine sichere mechanische Halterung der Eintauchsonde 5 in jedem praktisch denkbaren Fall möglich.

## Patentansprüche

1. Vorrichtung zur Durchführung von Messungen und/oder Probennahmen in Metallschmelzen mit einer Sublanze (4), die einen Sublanzenkörper aufweist, an dessen einem Ende ein Lanzenhalter zur Aufnahme einer Eintauchsonde (5) angeordnet ist, wobei an dem Lanzenhalter ein Kontaktstück (7) angeordnet ist zur Kontaktierung von Signalleitungen der Eintauchsonde (5), **dadurch gekennzeichnet dadurch, dass** der Sublanzenkörper mit dem Lanzenhalter beweglich verbunden ist und der Lanzenhalter mehrere gegeneinander bewegliche Teile aufweist, wobei das Kontaktstück (7) beweglich zum Sublanzenkörper angeordnet ist.

2. Vorrichtung nach Anspruch 1, **dadurch gekennzeichnet, dass** der Sublanzenkörper mit dem Lanzenhalter und/oder die Teile des Lanzenhalters in Achsrichtung und/oder radial beweglich angeordnet sind.

3. Vorrichtung nach Anspruch 2, **dadurch gekennzeichnet, dass** die axiale Beweglichkeit und die radiale Beweglichkeit durch voneinander verschiedene Paare von Verbindungsteilen realisiert sind.

4. Vorrichtung nach einem der Ansprüche 1 bis 3, **dadurch gekennzeichnet, dass** die Beweglichkeit durch zwischen starren Teilen angeordnete elastische Teile realisiert ist.

5. Vorrichtung nach Anspruch 4, **dadurch gekennzeichnet, dass** die elastischen Teile als Schraubenfeder oder elastischer Ring ausgebildet sind.

6. Vorrichtung nach einem der Ansprüche 1 bis 5, **dadurch gekennzeichnet, dass** ein Teil des Lanzenhalters eine Aufnahmebohrung aufweist, in der ein Zapfen eines zweiten Teils des Lanzenhalters axial beweglich angeordnet ist, wobei zwischen dem ersten und dem zweiten Teil des Lanzenhalters eine Schraubenfeder (14) angeordnet ist.

7. Vorrichtung nach Anspruch 6, **dadurch gekennzeichnet, dass** die Schraubenfeder zwischen einer an der Stirnseite oder an der Umfangsfläche des Zapfens angeordneten Anschlagsfläche und einer an oder in der Bohrung angeordneten Anschlagsfläche angeordnet ist.

8. Vorrichtung nach einem der Ansprüche 1 bis 7, **dadurch gekennzeichnet, dass** ein Teil des Lanzenhalters eine Aufnahmebohrung aufweist, in der ein Zapfen eines zweiten Teils des Lanzenhalters radial beweglich angeordnet ist, wobei zwischen dem ersten und dem zweiten Teil des Lanzenhalters mindestens ein elastischer Ring (11) angeordnet ist.

9. Vorrichtung nach Anspruch 8, **dadurch gekennzeichnet, dass** der elastische Ring (11) in radialer Richtung zwischen den beiden Teilen des Lanzenhalters angeordnet ist.

## Claims

1. Device for carrying out measurements and/or taking samples in molten metals, comprising a sub-lance (4) having a sub-lance body, on the one end of which a lance holder is arranged for receiving an immersion probe (5), wherein a contact piece (7) is arranged on the lance holder for contacting signal lines of the immersion probe (5), **characterised in that** the sub-lance body is moveably connected to the lance holder and the lance holder has a plurality of parts which can be moved relative to one another, the contact piece (7) being moveably arranged with respect to the sub-lance body.

2. Device according to claim 1, **characterised in that** the sub-lance body with the lance holder and/or the parts of the lance holder are arranged so as to be moveable in the axial direction and/or radially.

3. Device according to claim 2, **characterised in that** the axial mobility and the radial mobility are implemented by pairs of connection parts that are different from one another.

4. Device according to any one of claims 1 to 3, **characterised in that** the mobility is produced by elastic parts arranged between rigid parts.

5. Device according to claim 4, **characterised in that** the elastic parts are configured as a helical spring or as an elastic ring.

6. Device according to any one of claims 1 to 5, **characterised in that** a part of the lance holder has a receiving bore, in which a pin of a second part of the lance holder is arranged so as to be axially moveable, a helical spring (14) being arranged between the first and the second part of the lance holder.

7. Device according to claim 6, **characterised in that** the helical spring is arranged between a stop face arranged at the end face or on the peripheral face of the pin and a stop face arranged on or in the bore.

8. Device according to any one of claims 1 to 7, **characterised in that** a part of the lance holder has a receiving bore, in which a pin of a second part of the lance holder is radially moveably arranged, at least one elastic ring (11) being arranged between the first and the second part of the lance holder.

9. Device according to claim 8, **characterised in that** the elastic ring (11) is arranged in the radial direction between the two parts of the lance holder.

## Revendications

1. Dispositif pour effectuer des mesures et/ou des prélèvements d'échantillons dans des bains de fusion avec une sublance (4), qui présente un corps de sublance, à une extrémité duquel est disposé un support de lance pour recevoir une sonde d'immersion (5), où un élément de contact (7) est disposé dans le support de lance pour venir en contact de lignes signal de la sonde d'immersion (5), **caractérisé en ce que** le corps de sublance est relié mobile au support de lance et le support de lance présente plusieurs parties mobiles les unes par rapport aux autres, où l'élément de contact (7) est disposé mobile par rapport au corps de sublance.

2. Dispositif selon la revendication 1, **caractérisé en ce que** le corps de sublance est disposé mobile dans le sens axial et/ou radial avec le support de lance et/ou les parties du support de lance.

3. Dispositif selon la revendication 2, **caractérisé en ce que** la mobilité axiale et la mobilité radiale sont réalisées par une paire de parties de liaison différentes l'une de l'autre.

4. Dispositif selon l'une des revendications 1 à 3, **caractérisé en ce que** la mobilité est réalisée par des parties élastiques disposées entre les parties rigides.

5. Dispositif selon la revendication 4, **caractérisé en ce que** les parties élastiques sont réalisées sous la forme de ressorts à boudin ou bague élastique.

6. Dispositif selon l'une des revendications 1 à 5, **caractérisé en ce qu'**une partie du support de lance présente un alésage de réception, dans lequel un tourillon d'une seconde partie du support de lance est disposé mobile axialement, dans lequel un ressort à boudin (14) est disposé entre la première et la seconde partie du support de lance.

7. Dispositif selon la revendication 6, **caractérisé en ce que** le ressort à boudin est disposé entre une surface de butée disposée sur le côté antérieur ou sur la surface périphérique du tourillon et une surface de butée disposée sur ou dans l'alésage.

8. Dispositif selon l'une des revendications 1 à 7, **caractérisé en ce qu'**une partie du support de lance présente un alésage de réception, dans lequel un tourillon d'une seconde partie du support de lance est disposé mobile radialement, dans lequel au moins une bague élastique (11) est disposée entre la première et la seconde partie du support de lance.

9. Dispositif selon la revendication 8, **caractérisé en ce que** la bague élastique (11) est disposée dans le sens radial entre les deux parties du support de lance.
